(19) Europäisches Patentamt
European Patent Office
Office européen des brevets

(11) Numéro de publication: **0 345 153**
**A1**

(12) **DEMANDE DE BREVET EUROPEEN**

(21) Numéro de dépôt: **89401484.4**

(22) Date de dépôt: **31.05.89**

(51) Int. Cl.[4]: **C 07 J 51/00**
A 61 K 49/02, A 61 K 43/00,
C 07 B 59/00

(30) Priorité: **31.05.88 FR 8807222**

(43) Date de publication de la demande:
**06.12.89 Bulletin 89/49**

(84) Etats contractants désignés:
**AT BE CH DE ES FR GB GR IT LI LU NL SE**

(71) Demandeur: **IRE-CELLTARG S.A.**
**Zone Industrielle**
**B-6220 Fleurus (BE)**

**CENTRE NATIONAL DE LA RECHERCHE SCIENTIFIQUE (CNRS)**
**15, Quai Anatole France**
**F-75007 Paris (FR)**

(72) Inventeur: **Jaouen, Gérard**
**15, allée du Parc de la Bièvre**
**F-94240 L'Hay Les Roses (FR)**

**Vessieres, Anne**
**15, allée du Parc de la Bièvre**
**F-94240 L'Hay Les Roses (FR)**

(74) Mandataire: **Warcoin, Jacques et al**
**Cabinet Régimbeau 26, avenue Kléber**
**F-75116 Paris (FR)**

(54) **Marqueurs d'affinités de récepteurs d'hormones stéroides application en hormonoradiothérapie et comme agents d'imagerie des cancers hormonodépendants.**

(57) L'invention a pour objet des marqueurs d'affinités consistant en un ligand spécifique et irréversible de récepteurs d'hormones stéroïdes, caractérisés en ce qu'ils consistent en un complexe d'oestrogène constitué d'un dérivé de l'oestradiol complexé par un composé organométallique fixé en position 17$\alpha$ de l'oestradiol, complexe d'oestrogène dont le $PK_R+$, qui mesure la constante d'équilibre entre l'alcool en 17$\beta$ et la forme carbénium en 17 dudit complexe, est négatif.

La présente invention a également pour objet l'application de ces marqueurs d'affinitifs comme agents d'imagerie de cancers hormonodépendants et comme médicament pour l'hormonoradiothérapie lorsque ces marqueurs d'affinités comportent des isotopes radioactifs convenablement choisis.

EP 0 345 153 A1

## Description

# MARQUEURS D'AFFINITES DE RECEPTEURS D'HORMONES STEROIDES, APPLICATION EN HORMONORADIOTHERAPIE ET COMME AGENTS D'IMAGERIE DES CANCERS HORMONODEPENDANTS

La présente invention concerne des composés appelés "marqueurs d'affinités" de récepteurs d'hormones stéroïdes, consistant en des ligands spécifiques et irréversibles desdits récepteurs d'hormones stéroïdes, ainsi que leur application comme médicament pour l'hormonoradiothérapie de cancers hormonodépendants et comme agents d'imagerie de ces cancers.

L'explosion récente de la chimie des complexes de métaux de transition en synthèse organique a tenu, pour une grande part, au fait que par complexation temporaire d'un ligand organique, on en modifiait les propriétés de façon à autoriser des réactions difficiles ou impossibles par des voies classiques. Selon l'invention, ce concept est appliqué à des problèmes d'intérêt biologique conduisant ainsi à des ouvertures encore inédites pour cette chimie.

On assiste actuellement à une redéfinition dans l'industrie des rôles de la chimie organométallique moléculaire dans la mesure où elle se lance sur des pistes précédemment inexplorées tant en biologie, agrochimie que pharmacologie (1). La notion de sélectivité est une idée fédératrice de ces nouvelles directions. L'idée de créer des marqueurs d'affinités efficaces et sélectifs par mise à profit des particularités de la chimie des complexes des métaux de transition (caractère général de la stabilisation des ions $\alpha$-carbéniums adjacents à un organométallique) n'a, cependant, jamais été explorée.

Les "marqueurs d'affinités" sont des molécules (analogues chimiquement réactifs d'un bioligand spécifique) capables de former une liaison covalente irréversible avec la protéine réceptrice sous examen (par exemple récepteurs des hormones stéroïdes). Les intérêts en sont divers. On citera, entre autres, la possibilité ainsi offerte, d'identifier, de purifier dans des conditions dénaturantes, de concentrer, voire d'isoler les macromolécules protéiques. Ajoutons que cette technique permet aussi en principe d'analyser les propriétés biologiques de la macromolécule si le complexe covalent ligand-récepteur demeure biologiquement actif et encore de localiser le site d'association du ligand, puis d'obtenir des informations quant à la composition en acides aminés du site ainsi marqué. Cette liste n'est pas limitative. En particulier, en fonction de la nature de la modification chimique portée par le ligand (présence d'isotopes radioactifs tels que $^{99}Tc$, $^{103}Ru$, $^{106}Ru$) le domaine d'application de cette approche s'étendra à l'imagerie médicale et à la thérapeutique selon le concept d'hormonoradiothérapie.

Le marquage doit être efficace et sélectif. Pour évaluer le degré de satisfaction à ces deux impératifs, on utilise d'abord des récepteurs partiellement purifiés contenus dans un cytosol préparé à partir d'un tissu cible comme l'utérus de brebis, puis des systèmes intacts (culture de cellules riches en récepteurs d'oestrogènes comme la souche MCF7).

Les approches pratiques du marquage covalent des récepteurs des stéroïdes se classent essentiellement en deux grandes catégories faisant appel soit à des fonctions électrophiles, soit à des entités photoréactives (2). Le marquage par photoaffinité est considéré comme supérieur sur le plan de la sélectivité. Cependant, l'efficacité de cette technique s'avère souvent à l'expérience extrêmement basse. Au contraire, les agents électrophiles peuvent marquer les récepteurs de façon tout à fait efficiente, mais au détriment de la sélectivité en raison d'une réactivité intrinsèque non contrôlée (2). On pourrait envisager de tourner cette difficulté en imaginant des approches où les fonctions impliquées dans l'association ligand-récepteur sont activées chimiquement dans une étape ultérieure (3).

Quelques exemples, illustrant les voies classiques, figurent ci-dessous :

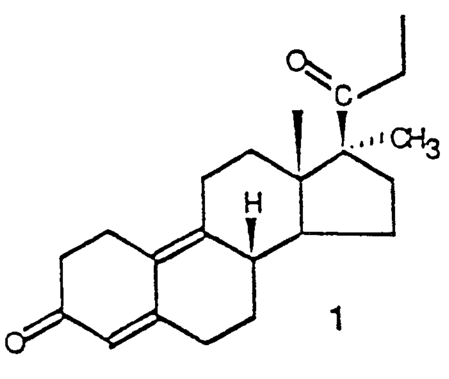

1

Récepteur de la progestérone :

1, R5020 activé photochimiquement

Marquage peu efficace (15 % après 1H) mais très sélectif.

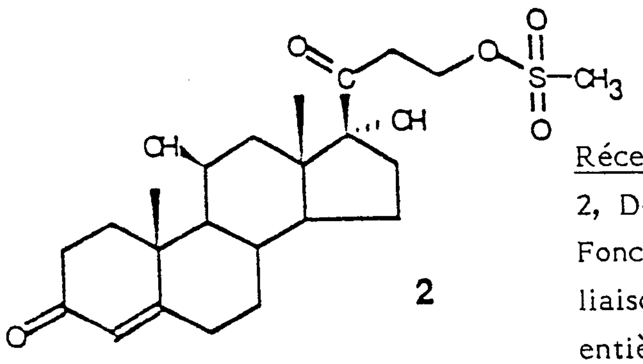

2

Récepteur des glucocorticoïdes :

2, Dexaméthasone-mésylate-21. Fonction électrophile. Taux élevé de liaison non-spécifique sur cellules entières. (8)

En série du récepteur de l'oestradiol, les deux types précédents d'approche classique ont aussi été illustrés, que ce soit la voie photochimique avec l'azide de l'hexestrol 3 (efficacité d'inactivation faible : 10-15%) (4) ou électrophile avec la tamoxifène aziridine 4 (inconvénient:anti-oestrogène) (5) et l'hexestrol aziridine 5 (6) difficiles à manipuler.

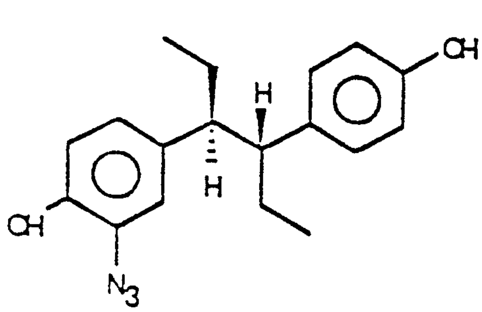

3

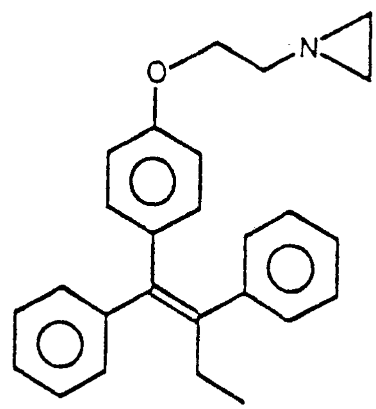

4

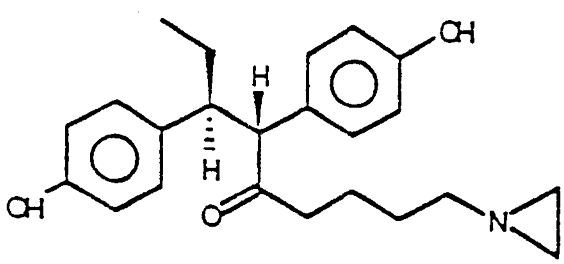

5

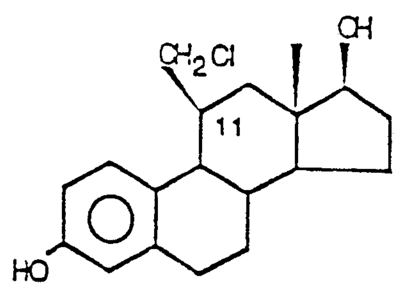

6

Il y a lieu de remarquer qu'il n'existe pas, à ce jour, de marqueur d'affinité du récepteur de l'oestradiol forgé à partir du squelette de l'oestradiol dans la mesure où le chlorométhyl 11β-oestradiol 6, un moment suspecté, a été démonté récemment comme n'en étant pas un (7).

Le but de la présente invention est donc de proposer des dérivés de l'oestradiol utilisables en marquage d'affinité efficace et sélectif dans le but, entre autres, de connaître le domaine de fixation de l'hormone, puis d'engendrer des anticorps anti-récepteurs (applicables au clonage des gènes correspondants) en s'appuyant sur une stratégie inédite faisant appel aux particularités de la chimie organométallique. A cet égard, il est intéressant de remarquer que ce marquage covalent par hormones organométalliques ressemble, en un sens, au comportement des substrats suicides d'enzymes qui ne deviennent sélectivement activés que dans le site actif des enzymes par des processus catalytiques spécifiques (10). Ajoutons enfin que la complexation d'hormones par métaux permet de nouveaux domaines d'applications, tant sur le plan structural, de l'imagerie

que de la thérapeutique pour peu que les éléments métalliques soient eux-mêmes bien choisis comme on l'a vu.

La présente invention a donc pour objet des marqueurs d'affinités consistant en des ligands spécifiques et irréversibles d'hormones stéroïdes, caractérisés en ce qu'ils consistent en un complexe d'oestrogène constitué d'un dérivé de l'oestradiol complexé par un composé organométallique fixé en position 17α de l'oestradiol, complexe dont le $PK_{R+}$, mesurant la constante d'équilibre entre l'alcool en 17β(ROH) et la forme carbénium en 17 ($R^+$) dudit complexe d'oestrogène, est négatif.

Le $PK_{R^+}$ mesure en effet la constante de l'équilibre

$$R^+ + H_2O \rightleftharpoons H^+ + ROH \qquad \text{avec} \quad K_{R^+} = \frac{[ROH][H^+]}{[R^+][H_2O]}$$

ROH représentant alors le complexe d'oestrogène dans son ensemble et OH étant la fonction OH en 17β avec $PK_{R^+} = -\log K_{R^+}$.

Ces complexes ROH que l'on peut aussi représenter par une formule de type

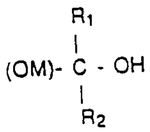 (OM = composé organométallique)

donnent facilement des ions carbéniums adjacents au composé organométallique. Ces ions ont un bon pouvoir alkylant. Celui-ci est d'autant meilleur que le $PK_{R^+}$ est négatif. Cette propriété est à l'origine de l'établissement du lien covalent du ligand stéroïde avec le récepteur comme il sera explicité par la suite.

On peut citer comme marqueurs d'affinités, selon l'invention, des ligands dans lesquels le composé organométallique consiste en un cluster alcyne complexé sur sa triple liaison répondant à la formule générale

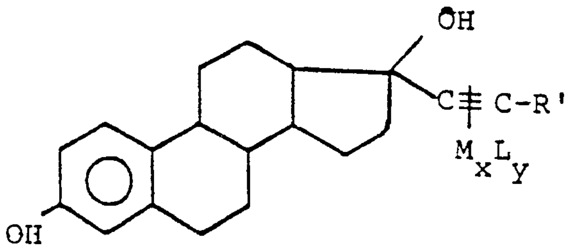

I

avec R′ qui est un hydrogène ou un groupe alkyle en $C_1$ à $C_7$ tel que $CH_3$ et

$M_xL_y$ qui représente le composé organométallique dans lequel

$M_x$ représente un ou plusieurs métaux de transition identiques ou différents et

$L_y$ représente un ou plusieurs ligands identiques ou différents complexant le ou lesdits métaux.

On peut citer comme autres marqueurs d'affinités, selon la présente invention, des ligands répondant à la formule générale

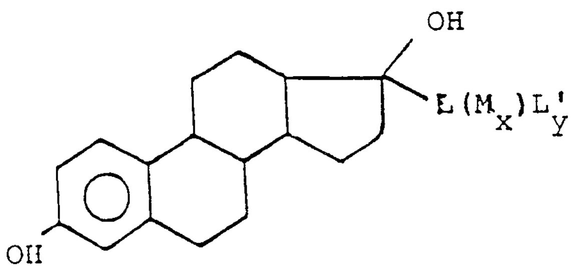

II

dans laquelle

$L(M_x)L'_y$ représente un composé organométallique où

$M_x$ a la signification donnée précédemment

L représente un ligand de $M_x$ lié à l'oestradiol en position 17α et $L'_y$ représente un ou plusieurs ligands identiques ou différents complexant le ou les métaux $M_x$.

On peut citer parmi les métaux appropriés du composé organométallique, selon l'invention, les métaux choisis parmi les groups VI, VII, VIII, et IX de la classification périodique des éléments.

Parmi les ligands de ces composés organométalliques, on peut citer à titre d'exemple CO, CS, CSe, CNR1, phényle, $P(R_2,R_3,R_4)$, cyclopentadiényle (Cp), $R_1$ étant notamment un radical alkyle ou $-COR_5$ et $R_2$, $R_3$, $R_4$ et $R_5$ étant notamment des radicaux phényle ou phénoxy substitué ou non, alkyle ou alcoxy en $C_1$ à $C_7$ substitué

ou non ou bien un atome d'halogène, $R_5$ pouvant être $-N(CH_2CH_2Cl)_2$.

Les composés $M_xL_y$ peuvent comporter plusieurs métaux et jusqu'à 12 ligands.

Comme marqueurs d'affinités de formule I, on peut citer ceux pour lesquels $M_xL_y$ est choisi parmi $Co_2(CO)_6$, $M_3(CO)_{10}$ avec M = Ru ou Os, et $Tc_2(CO)_{10}$.

Dans les marqueurs d'affinités de formule II, L′ est choisi de préférence parmi les groupes phényle et cyclopentadiényle.

On peut citer toujours dans ces marqueurs d'affinités de formule II les composés organométalliques suivants :

$(C_6H_5)Cr(CO)_3$, CpFeCp, $(C_6H_5)\overset{+}{T}c(CO)_3$, $(C_6H_5)\overset{+}{R}uCp$, $(C_6H_5)\overset{+}{F}eCp$, $(C_6H_5)\overset{+}{M}n(CO)_3$, CpRuCp.

Enfin, la présente invention a également pour objet des marqueurs d'affinités utiles à titre d'agents d'imagerie consistant dans les ligands décrits précédemment, caractérisés en ce que les métaux engagés dans les composés organométalliques sont sous forme d'isotopes métalliques radioactifs tels que du $Tc^{99}$, $Ru^{103}$ ou $Ru^{106}$.

La présente invention a également pour objet des marqueurs d'affinités utiles comme médicament pour l'hormonoradiothérapie de cancers hormonodépendants consistant dans les ligands spécifiques et irréversibles de récepteurs d'hormones stéroïdes, selon l'invention, caractérisés en ce qu'ils sont substitués par un groupe comportant au moins un isotope radioactif, tel qu'un iode 125 ou 123.

Les complexes d'hormones stéroïdes, selon l'invention, se lient aux récepteurs protéiques cytoplasmiques ou nucléaires de cellules cibles et, après liaison, subissent une translocation dans le noyau cellulaire et activent la transcription des portions du génome relatives à l'effet physiologique propre à l'hormone. Etant donné le passage du complexe stéroïde-récepteur à proximité du DNA, un iode radioactif $I^{125}$ ou $I^{123}$ porté par la stéroïde pourra endommager gravement le DNA et avoir un effet léthal sur la cellule cible.

Or un certain nombre de cancers présentent une concentration élevée en récepteurs spécifiques soit aux estrogènes, soit aux progestagènes, soit aux androgènes. Il s'agit notamment des cancers du sein, de l'utérus, de l'ovaire et de la prostate. Par exemple, 65 % des cancers du sein présentent des niveaux détectables de récepteurs d'estrogènes (de 5000 à 50000 molécules de récepteurs par cellule).

Un iode radioactif $I^{125}$ ou $I^{123}$ attaché au stéroïde permettra la destruction spécifique des cellules cancéreuses. En outre, un iode 123, comme les isotopes métalliques radioactifs, tels que $Tc^{99}$, $Ru^{103}$ ou $Ru^{106}$, permettra de visualiser la tumeur par radioimagerie. En fait, ces ligands constituent des agents de pilotage de l'élément actif proprement dit qui est leur radionucléide.

Les composés, selon la présente invention, peuvent être préparés par des procédés connus, notamment par action sur le composé oestrogène, ou le dérivé de composé oestrogène, du dérivé organo métallique correspondant. Bien entendu, lorsque cela sera nécessaire, certaines des fonctions du composé oestrogène pourront être protégées, notamment par silylation, là encore grâce à des procédés connus.

D'autres avantages et caractéristiques de la présente invention apparaîtront à la lumière des exemples qui vont suivre.

La figure 1 représente la cinétique d'inactivation du récepteur de l'oestradiol par les complexes 7 (■) et 8(□).

<u>EXEMPLE 1</u> : Ligands comportant un cluster alcyne - composé organométallique

Les fonctions importantes pour l'association des dérivés de l'oestradiol au récepteur de l'oestradiol (oestrophiline) sont les groupes hydroxy en positions -3 et -17β-. On sait, d'autre part, que la position 17α-est tolérante vis-à-vis des modifications structurales et préserve, dans une certaine mesure, les capacités de reconnaissance pour son récepteur de l'hormone ainsi modifiée (9). Ajoutons encore que l'hypothèse d'un site acide d'association du récepteur avec l'hormone a été formulée (5), que les ions carbéniums adjacents à un groupe organométallique sont très faciles à engendrer au départ d'alcools complexés et qu'ils ont largement démontré leur pouvoir alkylant (11). L'assemblage de tous ces composants fournit la base qui, selon l'invention, conduit à synthétiser des réactifs hormonaux dirigés spécifiquement sur le site actif du récepteur de l'oestradiol.

Dans cette optique, des composés tels que 7 et 8 sont préparés (9b). Ils portent des petits clusters en 17α- et couvrent une gamme de valeurs de $PK_{R^+}$ de -6 pour 7 à +3 pour 8. Ils ont servi de support à des études préliminaires de faisabilité car les valeurs d'affinité relative de liaison (A.R.L) de ces composés vis-à-vis du récepteur de l'oestradiol sont satisfaisantes (13 % pour 7 et 12 % pour 8).

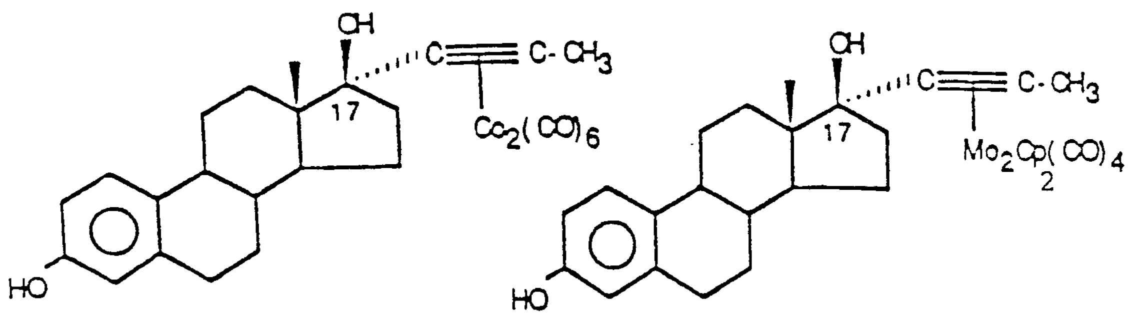

7 8

La nature irréversible de l'interaction du composé 7 avec le récepteur a été démontrée en incubant un cytosol d'utérus de brebis (préalablement filtré sur Millex-GV 0.22 µM) en présence de 10 nM de complexe 7 ou 8 à 25° C pendant des temps variables. A la fin du temps d'incubation, l'hormone libre, présente dans le milieu, est éliminée par traitement au charbon dextran. Les fractions de cytosol sont ensuite incubées à 20° C pendant 17 h. en présence de 10 nM d'oestradiol tritié. La fraction d'oestradiol tritiée liée (qui représente la quantité du récepteur qui n'a pas été marquée de façon covalente) est déterminée après précipitation des fractions liées au sulfate de protamine (9b). Les résultats obtenus sont rassemblés dans la figure 1.

On remarque sur cette figure, qu'avec le complexe du molybdène 8, la liaison reste totalement réversible alors qu'avec le complexe du cobalt 7, il y a apparition d'une liaison irréversible. A 25° C, on obtient un marquage rapide et efficace qui atteint 82 % après une heure d'incubation (moyenne de 4 expériences). L'efficacité du marquage dépend également de la température d'incubation : après 4 heures d'incubation en présence de 7, 29 % de la liaison est inactivée dans le cas d'une incubation à 0° C contre 50 % avec une incubation à 25° C (valeur moyenne de 3 expériences pour un cytosol non filtré). On a vérifié que cette liaison irréversible ne se fait pas si l'on pré-incube le cytosol en présence de 30 nM d'oestradiol non radioactif. L'ensemble de ces résultats indique donc la présence d'un marquage covalent du récepteur par le complexe organométallique 7. Il s'agit du premier type marqueur d'affinité du récepteur de l'oestradiol dont la structure possède le squelette de l'oestradiol.

Ces résultats s'étendent, bien sûr, à des molécules marquées (par organométallique et radioactivité), dont un exemple de synthèse est proposé schéma 1), réagissant avec des cellules entières et à d'autres systèmes organométalliques.

Schéma 1 : Préparation d'un marqueur d'affinité organométallique tritié.

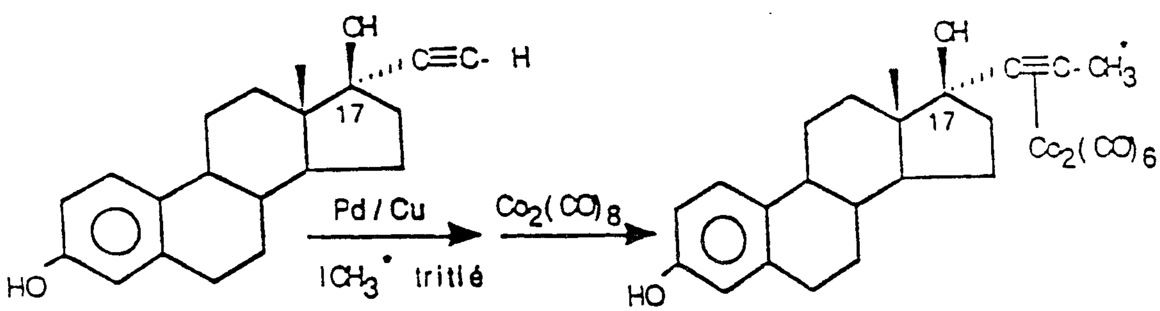


9 10

Les renseignements acquis donnent des indications sur la nature du site d'association hormone-récepteur. Les expériences sur les préparations cytosoliques montrent que la fonction alcool en 17β, modifiée par complexation en position adjacente, est activée sélectivement dans le site d'association du récepteur des oestrogènes, par acceptation d'un proton pour former une espèce réactive de type ion carbénium. Cette dernière agit alors comme agent alkylant d'un nucléophile voisin.

Il est intéressant de constater que l'ADN codant pour le récepteur de l'oestradiol a été cloné et sa séquence complète établie (12). Environ deux cents acides aminés du côté de la terminaison acide carboxylique du récepteur constituent le domaine dans lequel l'association avec le stéroïde est supposée s'établir.

On remarque dans le récepteur de l'oestradiol du poulet (mais également chez l'homme) que ce domaine comprend seulement quatre cystéines dont une (411) est adjacente à une lysine (410), une autre (441) possède une lysine en positionβ(443), tandis que la deuxième cystéine (524) est flanquée en α, α′ de deux lysines (523, 525). Ces positions sont excellentes pour fonctionner avec ce système.

Bien plus, des études chimiques préliminaires ont montré que les thiols réagissent sur l'hormone 7 pour donner un complexe électrophile (c'est le seul réactif acide qui se comporte ainsi) qui réagit instantanément sur une amine primaire avec une stéréochimie 17β. Avec l'hormone 8 et le thiol, la réaction observée est uniquement l'élimination. Les différences de $PK_{R^+}$ entre ces complexes sont en accord avec ce comportement. On peut expliquer ainsi pourquoi le cluster du molybdène 8 n'est pas un marqueur d'affinité.

Dès lors, une première ébauche du fonctionnement de marqueur est indiquée ci-dessous :

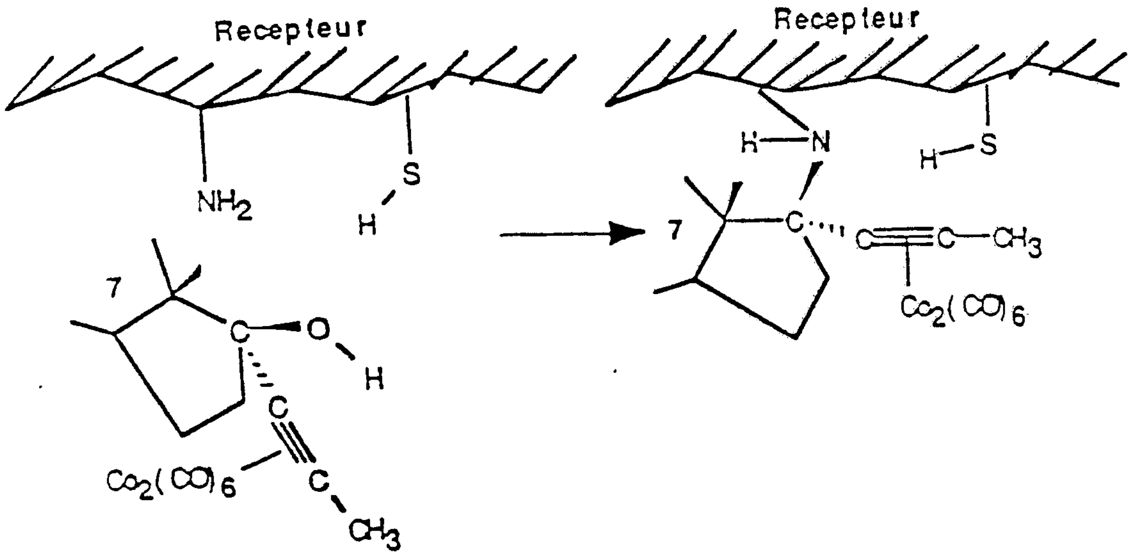

Il s'agit bien sûr de préciser et prouver ce mécanisme, de trouver le site exact d'association du récepteur avec la position 17β de l'oestradiol (par des analyses de séquences de fragments de récepteurs obtenus après protéolyse d'une préparation semi-purifiée du récepteur (13).

Préparation (éthynyl oestradiol) $Co_2(CO)_6$ (7')

Sous atmosphère d'argon, on ajoute doucement 0,6 g d'éthynyl oestradiol ($2.10^{-3}$ mole) dissous dans 20 ml d'éther anhydre dans une solution de 1,05 g de $CO_2(CO_8)$ ($3.10^{-3}$ mole) dans 10 ml d'éther. On laisse la réaction se poursuivre pendant 1 heure, puis on filtre et évapore le solvant. Le produit brut obtenu est purifié sur colonne de gel de silice Merck 8395 avec éluant E/Ep : 1/1. On obtient finalement 1 g de produit désiré, solide rouge sang. Il est difficile de donner un point de fusion exact à cause de la décomposition du produit. RMN ($CDCl_3$) cycle δ = 6,63 d (1), 6,70 dd (1),
7,23 d (1) ; 3 - OHδ = 6,21 s (1) ; $CH_3$ δ = 1,1 (3)

Analyse : $C_{26}H_{24}CO_2O_8$,

| | | | |
|---|---|---|---|
| trouvé | C 53,80 ; | H 4,61 ; | CO 19,70 |
| calculé | C 53,62 ; | H 4,15 ; | CO 20,24. |

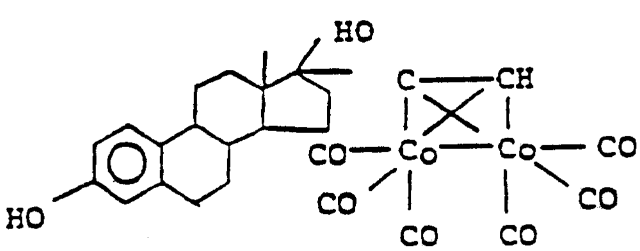

(7')

EXEMPLE 2 : Liaison irréversible entre le 17α-Ferrocényl-17β-oestradiol et les récepteurs nucléaires de l'oestradiol d'utérus de brebis

a) Préparation du 17α-Ferrocényl-17β-oestradiol

L'introduction du groupe ferrocénique se fait par addition du ferrocène lithien sur l'oestrone.

Le Ferrocène lithien est obtenu à partir du chloromercuriferrocène selon le schéma

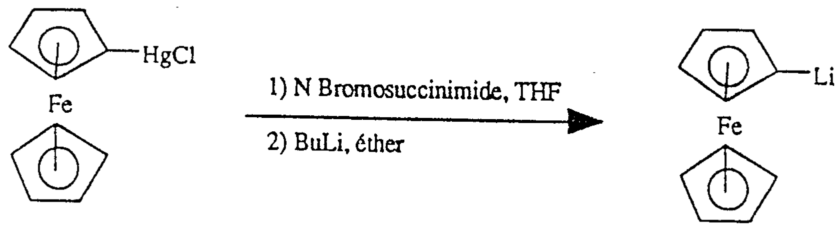


11

La réaction d'addition est :

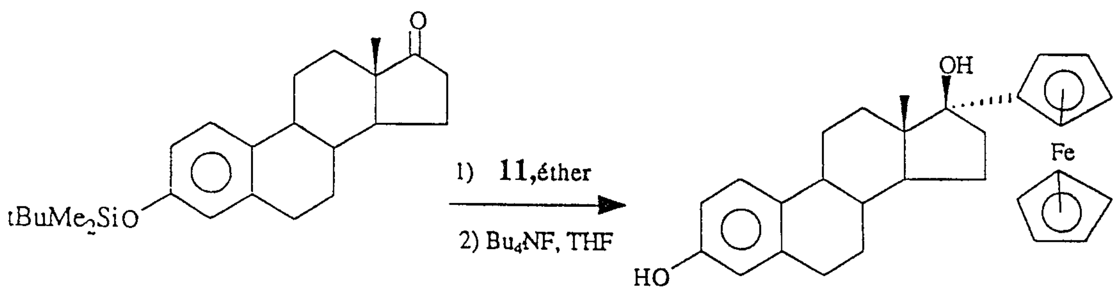


12

Partie expérimentale :

1. Silylation de l'oestrone de départ :

Dissoudre 1 g d'oestrone (3.7 $10^{-3}$ mol) dans 5 ml de DMF sec. Ajouter 613 mg de diméthylterbutylchlorosilane (4.07 $10^{-3}$ mol ; 1.1 eq) et 504 mg d'imidazole (7.4 $10^{-3}$ mol ; 2 eq). Laisser agiter à température ambiante pendant une heure à l'abri de l'humidité.

Un précipité blanc apparaît. Le dissoudre dans 50 ml d'éther et laver par 4 x 50 ml d'eau. Sécher sur $MgSO_4$ et évaporer à sec.

On recueille 1.1 g de produit cristallisé blanc. Rdt : 77 %.

2. Préparation du ferrocène lithien :

2.1 Bromation du ferrocène :

Préparer une solution de 575 mg de N bromosuccinimide 3.2 $10^{-3}$ mol) dans 50 ml de DMF sec, sous argon. Additionner goutte à goutte à 1.05 g de chloromercuriferrocène (2.49 x $10^{-3}$ mol) en solution dans 25 ml de DMF, à froid. Maintenir à 0°C pendant 3 heures. La solution est verte.

Ajouter 100 ml d'une solution de thiosulfate de sodium à 10 % et verser dans un litre d'eau froide. Extraire la phase aqueuse par 4 x 50 Ml d'éther. Sécher et évaporer à sec.

On recueille 496 mg de bromoferrocène (sous forme de cristaux marron) ; F = 35°C Rdt : 75 %.

2.2 Lithiation :

Préparer une solution de butyllithium d'environ 1.5 M, à partir de lithium et de bromobutane dans l'éther. Titrer précisément la solution.

Dissoudre 435 mg de bromoferrocène (1.64 x $10^{-3}$ mol) dans 3 ml d'ether sec. Ajouter 2 eq de butyllithium en solution dans l'éther. Laisser agiter quelques secondes à température ambiante, sous argon. La solution est rouge.

3. Addition sur l'oestrone silylée :

Refroidir la solution de ferrocène lithien précédemment obtenu à -50°C. Ajouter, par un goutte à goutte rapide 630 mg d'oestrone silylée (1.64 x $10^{-3}$ mol) dissous dans 20 ml d'éther et 5 ml de THF. Laisser agiter un quart d'heure. Ajouter alors 100 ml d'eau pour hydrolyser. Laver la phase organique à l'eau distillée, sécher sur $MgSO_4$, évaporer à sec et séparer par chromatographie sur plaques (éluant : éther/pentane 1/4). Deux produits sont présents : le ferrocène (Rf = 0.9) et le complexe recherché (Rf = 0.7).

On recueille 270 mg de produit jaune. Rdt : 29 %.

4. Déprotection :

Dissoudre les 270 mg de produit précédemment obtenu dans 5 ml de THF. Ajouter alors 0.5 ml de fluorure de tétrabulyammonium en solution dans le THF. Agiter quelques minutes. Hydrolyser et extraire par 4 x 20 ml

de $CH_2Cl_2$. Sécher et évaporer à sec. Laver au pentane et recristalliser dans un mélange dichlorométhane/ pentane.
$^1H$ RMN ($CDCl_3$, ppm) 1,0 (s) : Me-13 ; 7,03 (d) : $H_1$ ; 6,58 (dd) ; $H_2$ ; 6,53 (dd) : $H_4$ ; 2,77 : $H_6$ (m) ; 4,02 ; 4,16 ; 4,24 ; 4,29 : $C_5H_5$
* Chromatographie :
éluant : éther/pentane 1/1 Rf = 0.5

* Microanalyse :

| | | |
|---|---|---|
| calculé (%) | C 73.68 | H 7.02 |
| trouvé | C 73.75 | H 7.11 |

* Fusion (banc Koffler) : 175° C* Spectographie de masse :
pic de masse : 456
pic de base : 186 $Fe(C_5H_5)^{2+}$
* Infrarouge (pastille de KBr) :
v(CH) 3400, 2927, 2866, 816 $cm^{-1}$
v(CC) 1500, 1456, 1382, 1104, 1101 $cm^{-1}$
* UV (dans l'éthanol) : 228 nm $\varepsilon = 8300$
278 nm $\varepsilon = 3500$
* Voltammétrie cyclique : Ferrocène/ferricinium
E 1/2 = 0.515 V

b) Mise en évidence d'une liaison irréversible entre le [17α-Ferrocényl]-17β-oestradiol et les récepteurs nucléaires de l'oestradiol d'utérus de brebis

La nature irréversible de l'intéraction du composé 12 avec le récepteur a été démontrée en incubant un extrait nucléaire d'utérus de brebis en présence de molarités variables de complexe 12. A la fin du temps d'incubation, l'hormone libre, présente dans le milieu, est éliminée par traitement au charbon dextran. Les fractions d'extrait nucléaire sont ensuite incubées à 20° C pendant 17 h. en présence de 10 nM d'oestradiol tritié. La fraction d'oestradiol tritiée liée (qui représente la quantité de récepteur qui n'a pas été marquée de façon covalente) est déterminée après précipitation des fractions liées au sulfate de protamine (9b). Les résultats sont rassemblés dans le tableau suivant

| Molarité d'incubation de 12 | % d'inhibition de la liaison de l'oestradiol |
|---|---|
| 10 nM | 81 % |
| 5 nM | 71 % |
| 2 nM | 50 % |

Ces résultats démontrent clairement que le 17A-Ferrocényl]-17β-oestradiol est capable de marquer de façon irréversible les récepteurs de l'oestradiol contenu dans un extrait nucléaire, l'efficacité de marquage est de 81 % pour une molarité de marqueur de 10 nM.

EXEMPLE 3 : Extension du concept à d'autres systèmes organométalliques

1) On a vu que le marquage d'affinité par voie de complexes est efficace avec des systèmes de type propargyl coordinés par $Co_2(CO)_6(PK_{R^+} = -6)$ et inexistant avec des greffons culsters de type $Mo_2(Cp)_2(CO)_4(PK_{R^+} = +3)$.Ce dernier cas constitue la valeur la plus élevée de $PK_{R^+}$ connue à ce jour dans cette chimie. Il fait donc office debutée. Le $PK_{R^+}$, compatible avec un marquage, est ainsi de préférence négatif. Des complexes à ligands organiques autres que les alcynes tels que la série $(C_6H_5)Cr(CO)_3(PK_{R^+} = -11,3$ pour le cation benzyle complexé) ou ferrocénique ($PK_{R^+}$ de $CpFeCpCH_2^+ = -1,5$) sont donc appropriés selon l'invention. L'étude d'autres systèmes organométalliques présente donc des particularités intéressantes. Quelques-unes figurent ci-après :

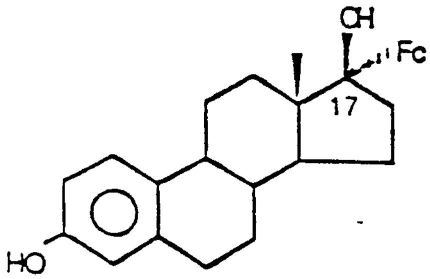

Fc = CpFeCp-, ferrocène
Bonne valeur d'ARL : 13 %
Passage aisé à l'ion ferricinium $Fc^{+}$ qui se comporte comme un alkylant doux et possède des propriétés antitumorales.

On peut effectuer des réactions d'échanges entre les métaux comme selon (14) :
Ferrocène + $^{103}RuCl_3 \longrightarrow$ [$^{103}$Ru]Ruthenocène + $FeCl_3$
ou d'autres réactions selon (15) comme :

$$^{103,106}RuCl_3 \xrightarrow[TiCl_3.H_2,C\bar{p},N\overset{+}{a}]{} [^{103,106}Ru]\text{Ruthenocène (CpRuCp)}$$

On voit ainsi la possibilité d'accéder aux séries des hormones du ferrocène et du ruthénium à des fins thérapeutiques et d'imagerie.

2) Une autre molécule de base, pour travailler avec du Cr, Mn, Tc et des complexes cationiques du Ruthénium (Ru) figure sur le schéma ci-dessous, a été synthétisée. Sa valeur d'A.R.L. vis-à-vis du récepteur de l'oestradiol est de 25 % :

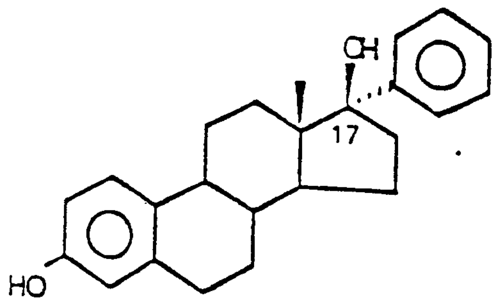

On sait aussi que les complexes de $(C_6H_5-)Cr(CO)_3$, $(C_6H_5-)\overset{+}{Mn}(CO)_3$ (l'équivalent avec $(C_6H_5-)\overset{+}{Tc}(CO)_3$ est possible, car $Tc_2(CO)_{10}$ a été décrit), $(C_6H_5-)\overset{+}{Fe}C_5H_5$, $(C_6H_5-)\overset{+}{Ru}C_5H_5$ existent. Il faut et suffit d'adapter les synthèses au cas particulier de l'hormone ci-dessus. La synthèse de $(C_6H_5-R)\overset{+}{Ru}(C_5H_5),PF_6^-$, par exemple peut se schématiser ainsi :

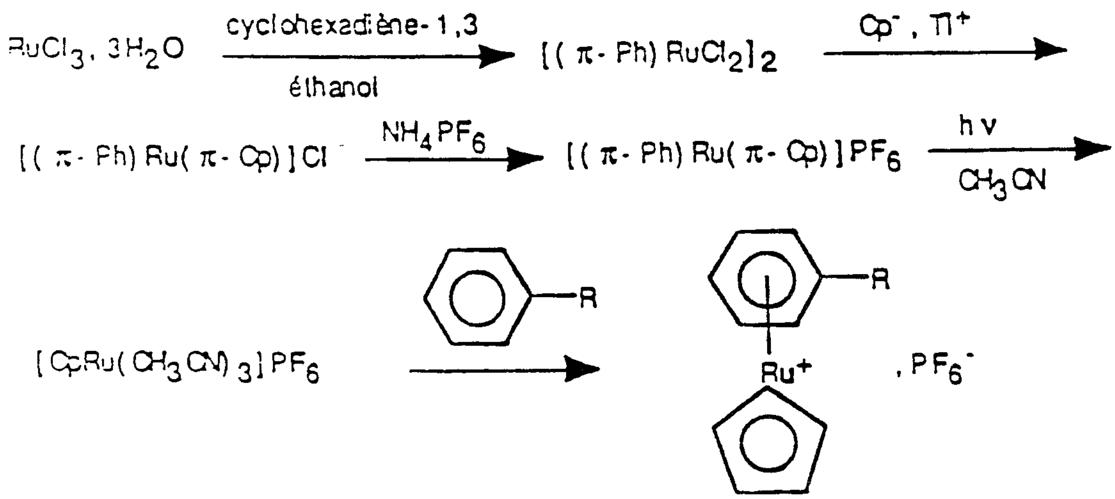


Remarque : le chlorure de [$^{103,106}$Ru] Ruthénium III est disponible commercialement.

Préparation du [17α-$C_6H_5$]-17β-oestradiol

L'oestrone est d'abord transformé en 3-O-(t.butyldiméthylsilyl)oestrone en faisant réagir le t.butyldiméthylchlorosilane avec l'oestrone en présence de NaH dans THF.

4 ml de phényllithium en solution dans benzène/éther (8 mmole) sont versés dans 30 ml de THF. Le mélange est refroidi à -78°C avec un bain acétone/carboglace. On ajoute ensuite goutte à goutte la solution de 3-O-(t.butyldiméthylsilyl)oestrone (0,61 g, 1,6 mmole) dans 30 ml de THF dans le shlenk contenant le phényllithium. L'addition dure 3 heures. La réaction continue pendant une nuit en laissant la température remonter doucement jusqu'à température ambiante. 5 gouttes de l'iodotriméthylsilane sont ajoutés pour enlever le groupe de protection. Au bout de 3 heures, on verse le milieu réactionnel dans une solution de $NH_4Cl$, on extrait le produit au $CH_2Cl_2$. Après séchage sur $MgSO_4$, filtration et évaporation, on obtient 0,67 g d'une huile incolore. La CCM révèle que la réaction n'est pas complète. Le produit brut réactionnel est chromatographié sur plaque de gel de silice avec comme éluant éther/pentane 1/1. On isole finalement 220 mg de (17 -$C_6H_5$)-17 -oestradiol, rendement : 39,5 %, solide incolore, F 225° (éther/pentane).

$^1$H RMN ($CD_3COCD_5$, ppm) : 7,90 : 3-OH (s) ; 7,46 (d), 7,46 (d), 7,3 (t), 7,21 (s) ; $C_6H_5$ ; 6,98 (d) : $H_1$ ; 6,53 (dd) : $H_2$ ; 6,49 (d) $H_4$ ; 2,75 (m) ; $H_6$ ; 1,08 (s) ; Me-13.

Analyse :

| | | | |
|---|---|---|---|
| Calc. : | C, 82,80 ; | H, 8,10 ; | O, 9,19. |
| Trouvé pour $C_{24}H_{28}O_2$ : | C, 81,85 ; | H, 8,13 ; | O, 9,31. |

Masse M/e : 348 ($M^+$), 330 ($M^+$-$H_2O$).

Préparation du [17α-$C_6H_5(Cr(CO)_3$][17β-oestradiol]

0,174 g de (17α-$C_6H_5$)-17β-oestradiol (0,5 mmole) et 0,44 g (2 mmole) de $Cr(CO)_6$ sont placés dans un ballon de 150 ml contenant 50 ml de l'éther dibutylique. On chauffe le mélange à ébullition douce pendant 3 h $\frac{1}{2}$. Après évaporation sous vide, le produit brut réactionnel obtenu est purifié sur plaque de gel de silice avec comme éluant éther/pentane : 3/2. On isole 70 mg du complexe, rendement : 29 %, solide jaune. La recristallisation dans éther/pentane fournit des cristaux jaunes, D° ≃ 204°C.

$^1$H RMN ($CD_3COCD_3$, ppm) : 7,97 (s) : 3-OH ; 7,02 (d). $H_1$ ; 6,56 (dd):$H_2$ ; 6,51 (d) ; 5,95 (d,1), 5,62 (m,4) : $C_6H_5$ ; 4,02 (s) : OH ; 2,76 (m) : $H_6$ ; 1,09 : $Me_{13}$.

Masse M/e : 484 ($M^+$), 428 ($M^+$-2CO), 400 ($M^+$-3CO), 384, 367, 348 ($M^+$-$Cr(CO)_3$), 330.

**BIBLIOGRAPHIE**


**1.** G.W. Parshall, Organometallics, 6, 687 (1987).

**2a.** H. Gronemeyer and M.V. Govindan, Mol. Cel. Endo. 46,1-19 (1987)

**2b.** J.A. Katzenellenbogen, B.S. Katzenellenbogen, Vitam. Horm. (N.Y.) 41, 213-274 (1984).

**3.**S.S. Simons, E.B. Thompson and D.F. Johnson, Biochemistry, 18, 4915-4922 (1979).

**4.** D.S. Robertson, L.L. Wei, J.R. Hayes, K.E. Carlson, J.A. Katzenellenbogen, B.S. Katzenellenbogen, Endocrinology, 109, 1298-1300 (1981).

**5.** J.A. Katzenellenbogen, K.E. Carlson, D.F. Heiman, D.S. Robertson, L.L. Wei, B.S. Katzenellenbogen, J.Biol. Chem., 258, 3487-3495 (1983).

**6.** J.A. Zablocki, J.A. Katzenellenbogen, K.E., Carlson, M.E., Norman, B.S. Katzenellenbogen, J.Med. Chem. 30, 829-838, (1987).

**7.** R.D.Bindal, Carlson, K.E., G.C.A. Reiner, J.A. Katzenellenbogen, J. Steroid Biochem. 28, 361-370 (1987).

**8.** S.S. Simons, E.B. Thompson, Proc. Natl. Acad. Sci. USA, 78, 3541-3545 (1981).

**9a** J.P. Raynaud, T. Ojasoo, M.M. Bouton, D. Philibert, Drug Design Vol VIII 169-214 (1979).

**9b** A. Vessières, G. Jaouen, Gruselle, M. Rossignol, J.L. Savignac, M. Top, S. Grenfield, J. Steroid Biochem., 29, 229-234 (1988).

**10** C.T. Walsh, Ann. Rev. Biochem., 53, 493-535 (1984).

**11** K.N. Nicholas, Accounts Chem. Res., 20, 207 (1987).

**12** A. Krust, S. Green, P. Argos, V. Kumar, P. Walter, J.M. Bornet, P. Chambon, EMBO J., 5, 891-897 (1986).

**13** S.S. Simons, J.G. Pumphrey, S. Rudikoff, H.J. Eisen J.Biol. Chem., 262, 9676-9680 (1987).

**14** M. Wenzel, P. Asindraza, G. Schachschneider, J. Labelled Comp. and Radiopharmaceuticals, Vol. XX, 1061-1071 (1983).

**15** W.H. Soine, C.E. Guyer, F.F. Knapp, J. Med. Chem. 27, 803-806 (1984).

**Revendications**

1. Marqueurs d'affinités consistant en un ligand spécifique et irréversible de récepteurs d'hormones stéroïdes, caractérisés en ce qu'ils consistent en un complexe d'oestrogène constitué d'un dérivé de l'oestradiol complexé par un composé organométallique fixé en position 17α de l'oestradiol, complexe d'oestrogène dont le $PK_{R^+}$, qui mesure la constante d'équilibre entre l'alcool en 17β et la forme carbénium en 17 dudit complexe, est négatif.

2. Marqueurs d'affinités selon la revendication 1, caractérisés en ce qu'ils consistent dans un ligand dans lequel le composé organométallique est constitué d'un cluster alcyne complexé sur sa triple liaison de formule générale

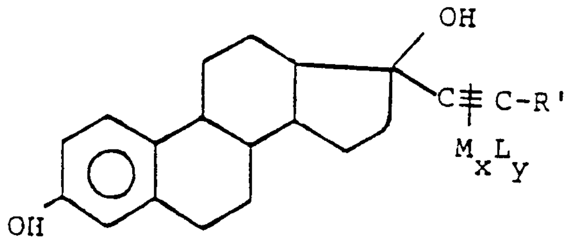

I

dans laquelle
R′ est un hydrogène ou un groupe alkyle en $C_1$ à $C_7$, notamment $CH_3$
$M_xL_y$ représente un composé organométallique complexant la triple liaison $C \equiv C$
$M_x$ représente un ou plusieurs métaux de transition identiques ou différents
$L_y$ représente un ou plusieurs ligands identiques ou différents complexant le ou les métaux.

3. Marqueurs d'affinités selon la revendication 1, caractérisés en ce qu'ils répondent à la formule générale II

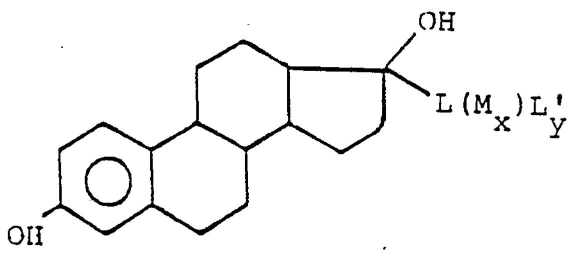

II

dans laquelle
$L(M_x)L'_y$ représente un complexe organométallique où $M_x$ a la signification donnée précédemment,
L représente un ligand de $M_x$ lié à l'oestradiol en 17α
$L'_y$ représente un ou plusieurs ligands identiques ou différents complexant $M_x$.

4. Marqueurs d'affinités selon l'une des revendications précédentes, caractérisés en ce que les métaux du composé organométallique sont choisis parmi les métaux des groupes VI, VII, VIII et IX de la classification périodique des éléments.

5. Marqueurs d'affinités selon l'une des revendications précédentes, caractérisés en ce que les ligands du complexe organométallique sont choisis parmi CO, CS, CSe, CNR1, phényle, $P(R_2,R_3R_4)$, cyclopentadiényle (Cp), $R_1$ étant notamment un radical alkyle ou $-COR_5$ et $R_2$, $R_3$, $R_4$ et $R_5$ étant notamment des radicaux phényle ou phénoxy substitué ou non, alkyle ou alcoxy en $C_1$ à $C_7$ substitué ou non ou bien un atome d'halogène, $R_5$ pouvant être $-N(CH_2CH_2Cl)_2$.

6. Marqueurs d'affinités selon l'une des revendications 2, 4 et 5, caractérisés en ce que $M_xL_y$ est choisi parmi
$Co_2(CO)_6$, $M_3(CO)_{10}$ avec M = Ru ou Os et $Tc_2(CO)_{10}$.

7. Marqueurs d'affinités selon l'une des revendications 3 à 5, caractérisés en ce que L est choisi parmi les groupes phényle et cyclopentadiényle.

8. Marqueurs d'affinités selon la revendication 7, caractérisés en ce que L $M_xL'_y$ est choisi parmi $(C_6H_5)Cr(CO)_3$, CpFeCp, CpRuCp, $(C_6H_5)\overset{+}{Tc}(CO)_3$, $(C_6H_5)\overset{+}{Ru}Cp$, $(C_6H_5)\overset{+}{Fe}Cp$, $(C_6H_5)\overset{+}{Mn}(CO)_3$.

9. Application des marqueurs d'affinités selon l'une des revendications précédentes, comme agents d'imagerie de cancers hormonodépendants, caractérisée en ce que les métaux du composé organométallique sont sous forme d'isotopes métalliques radioactifs, choisis notamment parmi le $Tc^{99}$, $Ru^{103}$ et $Ru^{106}$.

10. Application des marqueurs d'affinités selon l'une des revendications 1 à 8, comme agents d'imagerie

de cancers hormonodépendants, caractérisée en ce que le marqueur d'affinité est substitué par un groupe comportant un isotope radioactif, tel que $I^{123}$.

11. Application des marqueurs d'affinités selon l'une des revendications 1 à 8, à titre de médicament pour l'hormonoradiothérapie de cancers hormonodépendants, caractérisée en ce que le marqueur d'affinité est substitué par un groupe comportant un isotope radioactif, tel qu'un iode 125 ou 123.

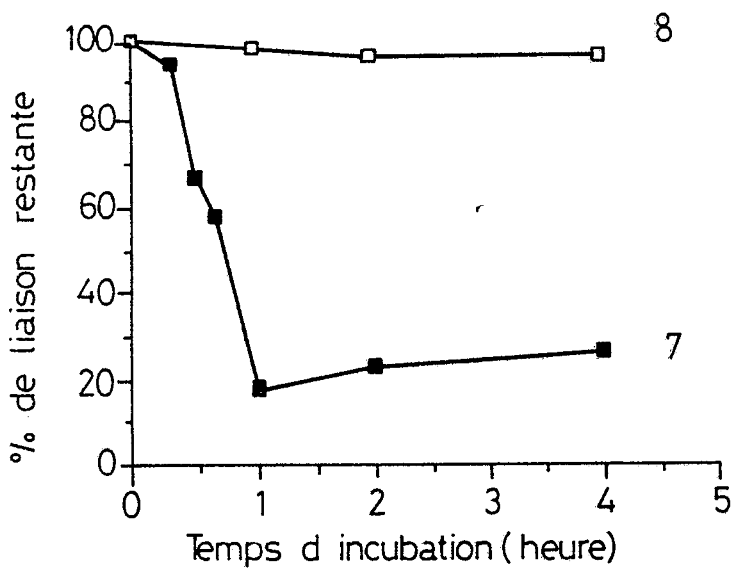
100
80
60
40
20
0
% de liaison restante
0
1
2
3
4
5
Temps d incubation ( heure)
8
7

FIG_1

Office européen des brevets

# RAPPORT DE RECHERCHE EUROPEENNE

Numero de la demande

EP 89 40 1484

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | PURE AND APPLIED CHEMISTRY, "Official Journal of the International Union of Pure and Applied Chemistry", vol. 57, no. 12, Décembre 1985, pages 1865-1871, Blackwell Scientific Publications, Oxford, GB; G. JAOUEN et al.: "Transition metal carbonyl oestrogen receptor assay" * En entier * --- | 1-11 | C 07 J 51/00<br>A 61 K 49/02<br>A 61 K 43/00<br>C 07 B 59/00 |
| X | ORGANOMETALLICS, vol. 6, no. 9, September 1987, pages 1985-1987, The American Chemical Society, Washington, D.C., US; G. JAOUEN et al.: "Transition-metal carbonyl clusters as novel infrared markers for estradiol receptor site detection" * En entier * --- | 1-11 | |
| A | EP-A-0 105 785 (C.N.R.S.) * Revendications * ----- | 1-11 | **DOMAINES TECHNIQUES RECHERCHES (Int. Cl.4)**<br>C 07 J 51/00<br>C 07 B 59/00 |

Le présent rapport a été établi pour toutes les revendications

| Lieu de la recherche | Date d'achèvement de la recherche | Examinateur |
|---|---|---|
| LA HAYE | 29-08-1989 | HENRY J.C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)